# EUROPEAN PATENT APPLICATION

(11) **EP 1 825 832 A1**
(43) Date of publication of application: **29.08.2007**
(21) Application number: 06425105.1
(22) Date of filing: 22.02.2006
(51) Int. Cl.: A61C 19/00, H01H 36/00, A61B 1/00, A61B 19/00

(54) **Medical apparatus**

(71) Applicant: CEFLA SOC. COOP. S.R.L., 40026 Imola (IT)
(72) Inventor: Nanni, Eros, 40023 Catel Guelfo di Bologna (IT); Virnicchi, Tommaso, 62019 Recanati (IT)
(74) Representative: Cerbaro, Elena

(57) **Abstract**

A medical apparatus is provided with an electrical circuit (17) for connecting together at least one powering battery (16) and a respective operating unit (10, 15), a magnetic switch (18) mobile between an open position and a closed position of the electrical circuit (17), a boxed container body (7) adapted to accommodate within the battery (16), the electrical circuit (17), and the magnetic switch (18), and a magnetic actuating device (19) arranged outside the boxed container body (7) for maintaining the magnetic switch (18) in its open position of the electrical circuit (17).

## Description

The present invention relates to a medical apparatus.

In particular, the present invention relates to a medical apparatus for dental use. In the discussion that follows, the medical apparatus for dental use taken into consideration is a polymerising lamp for polymerising dental compounds, to which explicit reference will be made in the present description without for this loosing in generality.

In the dental field, it is known the use of a photopolymerisable resin-based compound, which is applied in a semi-fluid state, and is then stabilised by means of a polymerising process triggered by radiating the compound itself with light at a determined wavelength.

The polymerisation process is triggered by means of a polymerising lamp of the type comprising optical means adapted to polymerise the mentioned dental compounds; logical control means adapted to selectively control the optical means; at least one battery for powering the mentioned logical control means; an electrical circuit for connecting the battery to the optical means and the logical control means; and a boxed container body adapted to accommodate within, in use, the optical means, the logical control means, the battery and the electrical circuit.

For safety reasons and to avoid deploying the battery, the battery must be disconnected from the electrical circuit at least before the first use of the polymerising lamp. This problem is currently solved by providing the user with a separate battery to be inserted within the polymerising lamp or to be fastened to an end of the boxed container body.

The first known solution of the type described above causes severe operative difficulties for the user, who must firstly open the boxed container body, then insert the battery, and finally close the container body again. The sequence of operations described above, if not performed correctly, may compromise correct operation of the polymerising lamp and furthermore may void the manufacturer's warranty.

The second solution of the known type described presents the severe drawback in that the polymerising lamp is longer, being imbalanced towards the supporting end of the battery, and therefore presents reduced ergonomics.

It is the object of the present invention to provide a medical apparatus which is free from the above mentioned drawbacks.

According to the present invention there is provided a medical apparatus as claimed in the attached claims.

The present invention will now be described with reference to the accompanying drawings illustrating a non-limitative embodiment example thereof, in which:
figure 1 is a schematic perspective view, with parts enlarged for clarity, of a preferred embodiment of the medical apparatus of the present invention; and
figure 2 is an exploded perspective view of a detail of the medical apparatus in figure 1.

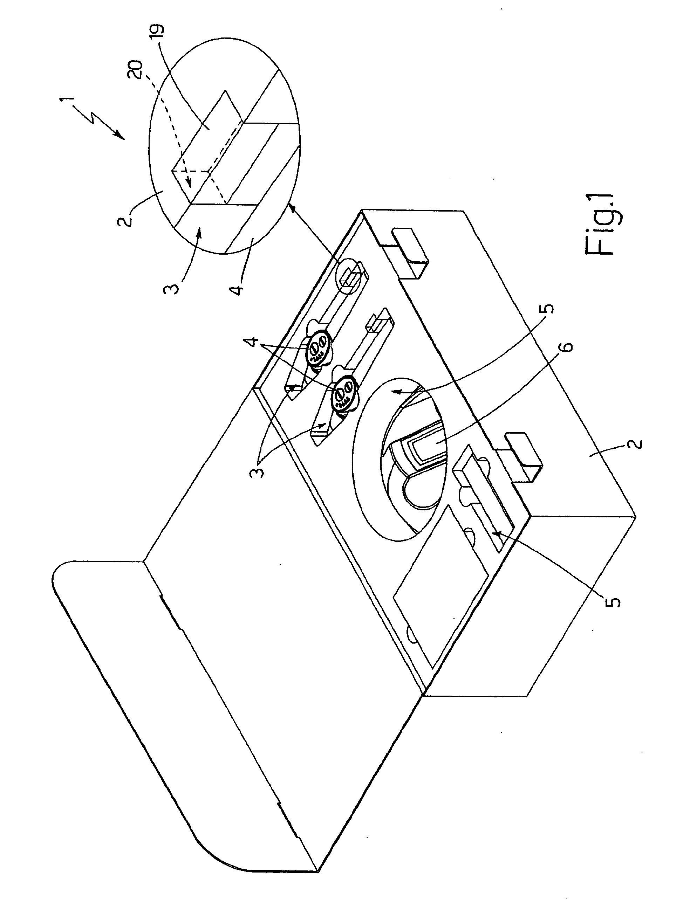

With reference to figure 1, it is indicated as a whole by 1 a medical apparatus comprising, in the case in point, a container 2 presenting two seats 3 for housing respective polymerising lamps 4 and a plurality of further seats 5 for housing some accessories including, for example, a battery charger 6.

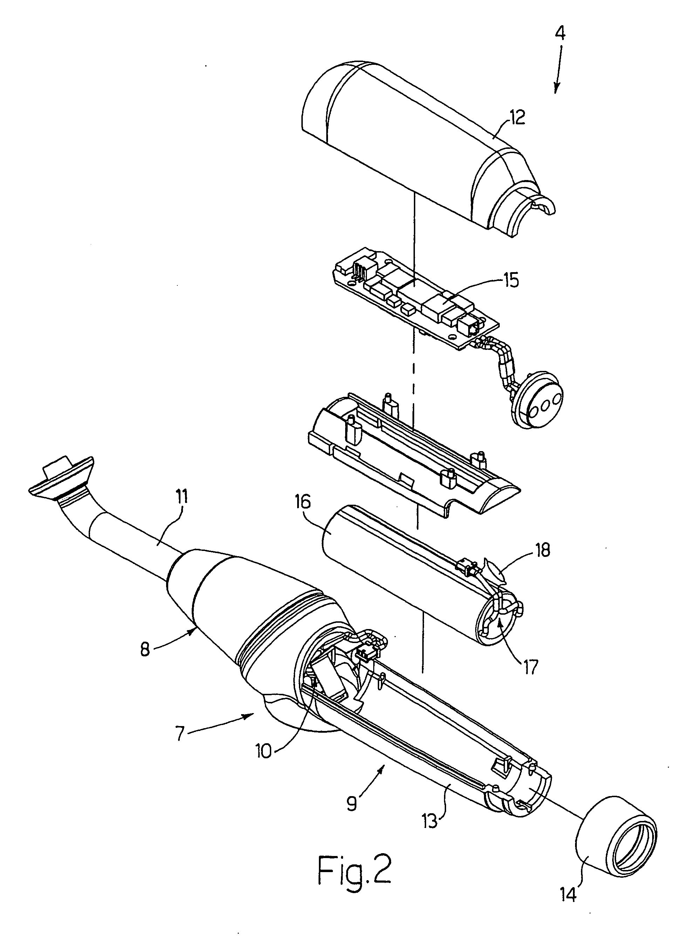

As shown in figure 2, each lamp 4 is adapted to polymerise compounds for dental use by radiation of the compounds themselves with light of a determined wavelength, and comprises a boxed container body 7 comprising, in turn, two housing elements 8, 9 reciprocally coupled in a known way.

The element 8 is a tubular element of essentially truncated cone shape, which supports an optical device 10 adapted to polymerise the mentioned dental compounds and comprising a light source of the known type and not shown arranged within the element 8 and an output optical fibre 11 of the known type, which is fastened to an end of the element 8 itself, is optically coupled to the mentioned light source (not shown), and is shaped so as to address the light output by the light source (not shown) itself to the oral cavity of a patient.

Element 9 is an essentially tubular body, which defines a grip of the lamp 4 and is defined by two essentially semi-cylindrical shells 12, 13 reciprocally coupled in the known way by means of a fastening ring nut 14.

Element 9 accommodates within a printed circuit 15 provided with electronic components for controlling the operation of the lamp 4, at least one battery 16, in the case in point a rechargeable battery, for powering the printed circuit 15 and the optical device 10, and an electrical circuit 17 for connecting the battery 16 to the printed circuit 15 and to the optical device 10.

The battery 16 and the electrical circuit 17 are selectively and reciprocally connected by means of a switch 18, in the case in point a magnetic switch, normally arranged in a closed position of the electrical circuit 17, in which the battery 16 electrically powers the printed circuit 15 and the optical device 10.

When the lamp 4 is arranged within the respective seat 3 of the container 2, the switch 18 is shifted to an open position of the electrical circuit 17, in which the battery 16 is disconnected from the printed circuit 15 and the optical device 10, by means of a magnetic actuator 19, in the case in point a permanent magnet, accommodated within a cavity 20 made in the container 2 in position facing the respective seat 3 and so as to allow the actuator 19 to operate the switch 18.

The medical apparatus 1 therefore presents the advantage that:
the polymerising lamps 4 are provided to the user with the respective batteries 16 already accommodated within the respective boxed container bodies 7;
the batteries 16 are disconnected from the respective printed circuits 15 and the optical devices 10 when the respective polymerising lamps 4 are arranged within the container 2; and
the batteries 16 are connected to the respective printed circuit 15 and the optical devices 10 without requiring any intervention by the user, without needing to open and close the respective shells 12 and 13, and only when the polymerising lamps 4 are extracted from the respective seats 3 in the container 2.

According to a variant (not shown), the container 2 is eliminated and each polymerising lamp 4 is provided with a magnetic actuator 19 removably fastened to the respective boxed container body 7 so that the detachment of the magnetic actuator 19 from the respective boxed container body 7 causes the shift of the respective switch 18 to its closed position.

According to a further variant (not shown), the polymerising lamps 4 are eliminated and replaced by different medical devices which entail the powering of an operating unit by at least one battery. In the dental field, the polymerising lamps 4 may be replaced, for example, by cameras.

## Claims

1. A medical apparatus comprising a medical device (4) comprising in turn an operating unit (10, 15), at least one battery (16) for powering said operating unit (10, 15), an electrical circuit (17) for reciprocally connecting the battery (16) and the operating unit (10, 15), a magnetic switch (18) mobile between an open position and a closed position of the electrical circuit (17) itself, and a boxed container body (7) adapted to accommodate within at least the battery (16), the electrical circuit (17), and the magnetic switch (18); and **characterised in that** it further comprises magnetic actuating means (19) arranged outside said boxed container body (7) to maintain the magnetic switch (18) in said open position.

2. A medical apparatus according to claim 1, wherein the medical device (4) and the actuating means (19) are reciprocally mobile between a close position, in which the actuating means (19) maintain the magnetic switch (18) in said open position, and a distanced position, in which the magnetic switch (18) is arranged in said closed position.

3. A medical apparatus according to claim 1 or 2 and further comprising a container (2) provided with a first seat (3) for accommodating said medical device (4) and a second seat (20) for accommodating said magnetic actuating means (19); the magnetic actuating means (19) being adapted to maintain the magnetic switch (18) in said open position when the medical device (4) is accommodated within the respective said first seat (3).

4. A medical apparatus according to claim 1 or 2, wherein said magnetic actuating means (19) are removably fastened to said container boxed body (7).

5. A medical apparatus according to any of the preceeding claims, wherein said battery (16) is a rechargeable battery.

6. A medical apparatus according to any of the claims from 1 to 4, wherein said battery (16) is a non-rechargeable battery.

7. A medical apparatus according to any of the preceeding claims, wherein said medical device (4) is a medical device for dental use.

8. A medical apparatus according to claim 7, wherein said medical device (4) is a polymerising lamp for polymerising compounds for dental use.

9. A medical apparatus according to claim 7, wherein said medical device (4) is a camera for dental use.
